# EUROPEAN PATENT APPLICATION

(11) **EP 2 186 520 A1**
(43) Date of publication of application: **19.05.2010**
(21) Application number: 08876782.7
(22) Date of filing: 11.11.2008
(51) Int. Cl.: A61K 31/711, A61K 31/198, A61K 31/375, A61P 21/00

(54) **THERAPEUTIC AGENT FOR FIBROMYALGIA SYNDROME AND THERAPEUTIC AGENT FOR PAIN DUE TO VASCULAR SMOOTH MUSCLE SPASM**

(30) Priority: 21.08.2008 JP 2008213345
(71) Applicant: Morishige, Fumie, Sanbu-gun Chiba 299-3236 (JP)
(72) Inventor: Morishige, Fumie, Sanbu-gun Chiba 299-3236 (JP)
(74) Representative: HOFFMANN EITLE
(86) International application number: PCT/JP2008/070511
(87) International publication number: WO 2010/021064

(57) **Abstract**

[TASK] It is an object to provide a therapeutic agent for fibromyalgia syndrome and a therapeutic agent for pain caused by vascular smooth muscle spasm.

[MEANS OF SOLVING THE PROBLEM] A therapeutic agent for fibromyalgia syndrome or therapeutic agent for pain caused by vascular smooth muscle spasm including
a) one or more compounds selected from a group consisting of RNA, ribonucleotide and ribonucleoside,
b) one or more compounds selected from a group consisting of L-arginine, L-citrulline and L-ornithine, and
c) L-ascorbic acid

in which the mass ratio of Component b) : Component c) is 1:0.2 to 20 is provided.

## Description

### TECHNICAL FIELD

The present invention relates to therapeutic agents for fibromyalgia syndrome and therapeutic agents for pain caused by vascular smooth muscle spasm including a) one or more compounds selected from a group consisting of RNA, ribonucleotide and ribonucleoside (hereinafter referred to as RNA and the like), b) one or more compounds selected from a group consisting of L-arginine, L-citrulline and L-ornithine, and c) L-ascorbic acid (hereinafter referred to as ascorbic acid) in which the mass ratio of Component b) : Component c) is 1:0.2 to 20, specifically 0.2 to 0.25.

### BACKGROUND ART

Chronic pain is a broad term generally defined as pain that continues over a usual time course of an acute disease or over a time for healing an injury, or pain that recurs every several months or every several years. The chronic pain has many symptoms, and examples of the symptom include a musculoskeletal or vertebral pain, neurological pain, headache or vascular pain.
Fibromyalgia syndrome classified as the chronic pain (hereinafter sometimes abbreviated as FMS) causes broad and long-lasting pain most complicatedly and has a feature of stronger perception relating to stimuli in whole body. The pain is diagnosed when eleven or more points are affected by pain among eighteen "tender points" upon palpation, and relates to various underlying diseases or disorders. Examples of other symptoms accompanied with the fibromyalgia syndrome include a depression, fatigue, sleep disorder and memory disorder.
Patients having fibromyalgia syndrome can be alleviated to some extent by an analgesic agent or antidepressant, or by adjunctive treatments such as moderate exercise, appropriate dietary therapy and stress release, but there is still no effective method for treating most of the fibromyalgia syndrome patients in current situation.

In Japanese Patent Application Publication No. JP-A-2004-089004 and Japanese Patent Application Publication No. JP-A-2003-335664, dietary supplements, nutritional preparations for cerebrospinal nerve and dietary supplements for complementary therapy including an RNA powder, L-arginine powder and L-Ascorbic acid powder are disclosed, and it is further described that addition of a certain amount of ascorbic acid can avoid a harsh taste of arginine and reduce a harsh feeling of arginine.
Furthermore, in International Publication WO 2005/084660 pamphlet, therapeutic agents for mitochondrial disease including RNA, L-arginine and L-ascorbic acid are described.
However, there are no reports in which compositions including RNA, L-arginine and L-ascorbic acid are used for treating fibromyalgia syndrome classified as chronic pain.
[Patent Document 1]
   Japanese Patent Application Publication No. JP-A- 2004-089004
[Patent Document 2]
   Japanese Patent Application Publication No. JP-A- 2003-335664
[Patent Document 3]
   International Publication WO 2005/084660 pamphlet

### DISCLOSURE OF THE INVENTION

### [Problem to be Solved by the Invention]

It is an object of the present invention to provide a therapeutic agent for chronic pain with excellent safety, and, in particular, a therapeutic agent for fibromyalgia syndrome and therapeutic agent for pain caused by vascular smooth muscle spasm.

### [Means for Solving the Problem]

The inventors of the present invention have carried out intensive studies in order to solve the above problem, and as a result, have found that a composition including RNA and the like, one or more compounds selected from a group consisting of L-arginine, L-citrulline and L-ornithine, and ascorbic acid has an excellent treatment effect on fibromyalgia syndrome.
Furthermore, since the composition also has an excellent treatment effect on fibromyalgia syndrome accompanied with spasm of muscle (for example eyelid), it is suggested that the composition has a treatment effect on pain caused by vascular smooth muscle spasm, furthermore, it is ascertained that the compound actually has an excellent treatment effect on the pain mentioned above (for example anginal pain), and the present invention has been accomplished.
The composition of the present invention including RNA and the like, one or more compounds selected from a group consisting of L-arginine, L-citrulline and L-ornithine, and ascorbic acid is extremely highly safe.

Specifically, the present invention relates to:
(1) a therapeutic agent for fibromyalgia syndrome including
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
   in which the mass ratio of Component b) : Component c) is 1:0.2 to 20,
(2) the therapeutic agent for fibromyalgia syndrome described in item (1) in which the mass ratio of Component b) : Component c) is 1:0.2 to 6,
(3) the therapeutic agent for fibromyalgia syndrome described in item (2) in which the mass ratio is 1:0.2 to 0.25,
(4) the therapeutic agent for fibromyalgia syndrome described in any one of items (1) to (3) in which one or more compounds selected from the group consisting of RNA, ribonucleotide and ribonucleoside are an extract(s) from yeast,
(5) the therapeutic agent for fibromyalgia syndrome described in item (4) in which the yeast is a brewer's yeast,
(6) the therapeutic agent for fibromyalgia syndrome described in any one of items (1) to (5) further including an extract derived from salmon soft roe,
(7) a therapeutic agent for pain caused by vascular smooth muscle spasm including
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
   in which the mass ratio of Component b) : Component c) is 1:0.2 to 20,
(8) the therapeutic agent for pain caused by vascular smooth muscle spasm described in item (7) in which the mass ratio of Component b) : Component c) is 1:0.2 to 6,
(9) the therapeutic agent for pain caused by vascular smooth muscle spasm described in item (8) in which the mass ratio is 1:0.2 to 0.25,
(10) the therapeutic agent for pain caused by vascular smooth muscle spasm described in any one of items (7) to (9) in which one or more compounds selected from the group consisting of RNA, ribonucleotide and ribonucleoside are an extract(s) from yeast,
(11) the therapeutic agent for pain caused by vascular smooth muscle spasm described in item (10) in which the yeast is a brewer's yeast,
(12) the therapeutic agent for pain caused by vascular smooth muscle spasm described in any one of items (7) to (11) further including an extract derived from salmon soft roe,
(13) use, for producing a therapeutic agent for fibromyalgia syndrome, of a composition including
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
   in which the mass ratio of Component b) : Component c) is 1:0.2 to 20,
(14) a method for treating fibromyalgia syndrome including
   administering a therapeutically effective amount of a composition containing
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
   to a patient requiring the treatment
   in which the mass ratio of Component b) : Component c) is 1:0.2 to 20,
(15) use, for producing a therapeutic agent for pain caused by vascular smooth muscle spasm, of a composition including
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
   in which the mass ratio of Component b) : Component c) is 1:0.2 to 20,
(16) a method for treating pain caused by vascular smooth muscle spasm including administering a therapeutically effective amount of a composition containing
   a) one or more compounds selected from a group consisting of RNA, ribonucleotide and ribonucleoside,
   b) one or more compounds selected from a group consisting of L-arginine, L-citrulline and L-ornithine, and
   c) L-ascorbic acid
to a patient requiring the treatment
in which the mass ratio of Component b) : Component c) is 1:0.2 to 20.

### [Effects of the Invention]

According to the present invention, a therapeutic agent for chronic pain with excellent safety, in particular, a therapeutic agent for fibromyalgia syndrome and a therapeutic agent for pain caused by vascular smooth muscle spasm can be provided.
As mentioned above, the diagnostic method of fibromyalgia syndrome is established, but the mechanism to cause fibromyalgia syndrome is not all clear. Accordingly, it is not clear why the composition including RNA and the like, one or more compounds selected from a group consisting of L-arginine, L-citrulline and L-ornithine, and ascorbic acid used in the present invention has an excellent treatment effect on fibromyalgia syndrome, but, for example, an independent effect of each component and/or an interactive effect of a plurality of components shown below are supposed to contribute multiply:
(a) RNA and the like are useful for a neurotrophic factor, and the availability of RNA and the like is accelerated by combination use with arginine.
(b) Intake of a large amount of L-arginine, L-citrulline and/or L-ornithine increases the concentration of GABA in neuronal cells that shows a sedative effect.
(c) Intake of a large amount of L-arginine, L-citrulline and/or L-ornithine generates kyotorphin (Tyr-Arg), which is a dipeptide showing an analgesic effect, to exert an inhibitory effect on pain.
(d) Intake of a large amount of L-arginine, L-citrulline and/or L-ornithine improves muscle spasm.
(e) Combination use of ascorbic acid eliminates abnormal occurrence of NOₓ due to the intake of a large amount of L-arginine, L-citrulline and/or L-ornithine to eliminate cell damages by NOₓ due to arginine.

Furthermore, pain caused by vascular smooth muscle spasm can be considered that the pain of muscle (skeletal muscle) in fibromyalgia syndrome is replaced by the pain of vascular muscle (smooth muscle), and thus, in the same manner as in fibromyalgia syndrome, for example, as a result of the various effects described above, excellent treatment effects are supposed to be shown.
In addition, the therapeutic agent of the present invention has treatment effects on not only the pain caused by vascular smooth muscle spasm but also pain caused by striated muscle spasm.
Furthermore, the therapeutic agent of the present invention has an effect to inhibit blood clot forming.

### BEST MODES FOR CARRYING OUT THE INVENTION

Each of the therapeutic agent for fibromyalgia syndrome and the therapeutic agent for pain caused by vascular smooth muscle spasm of the present invention includes
a) one or more compounds selected from a group consisting of RNA, ribonucleotide and ribonucleoside,
b) one or more compounds selected from a group consisting of L-arginine, L-citrulline and L-ornithine, and
c) L-ascorbic acid.

One or more compounds selected from a group consisting of RNA, ribonucleotide and ribonucleoside as Component a) mean RNA, ribonucleotide or ribonucleoside; RNA and ribonucleotide; RNA and ribonucleoside; ribonucleotide and ribonucleoside; and RNA, ribonucleotide and ribonucleoside.

One or more compounds selected from a group consisting of RNA, ribonucleotide and ribonucleoside (hereinafter referred to as RNA and the like) used in the present invention may be naturally occurring compounds or synthetic compounds. Examples of the naturally occurring RNA and the like include RNA and the like derived from fungi, preferably RNA and the like extracted and isolated from yeasts such as a brewer's yeast, baker's yeast, Torula yeast or milk yeast, and more preferably RNA and the like extracted from a brewer's yeast.

As for methods for obtaining naturally occurring RNA and the like, examples of the method for obtaining naturally occurring RNA and the like from a brewer's yeast include the following method. Namely, a residue removing a water soluble low molecular fraction from a slurry yeast (solid content: about 10%) or dry yeast that is recovered from a fabrication process of beer is used as a material. To the residue, a salt solution is added, RNA is extracted under heating, to the extraction liquid, a concentrated aqueous hydrochloric acid solution is added to precipitate. After neutralization RNA is reextracted with water, then an organic solvent with a poor solubility to RNA such as ethanol is added to precipitate RNA, and a supernatant is removed by centrifugation to obtain a precipitate fraction mainly containing RNA. Then, the precipitate fraction is dried by an appropriate usual procedure to obtain the objective RNA. A summary of the compositions of the dried brewer's yeast RNA obtained in this manner is shown below.

[Table 1]

**Table 1**

| Component | Content (on a dry basis) |
|---|---|
| RNA^{1*} | 65 to 95 (%) |
| CAS^{2*} | Less than 5 |
| DNA | Less than 1 |
| Protein | Less than 22 |
| Sugar (hexose) | Less than 18 |
| Sodium chloride | Less than 0.5 |

| | |
|---|---|
| 1* A molecular weight of about 1.3×10⁴ 2* Low-molecular-weight ribonucleic acid, ribonucleotide and ribonucleoside soluble in cold 5% perchloric acid. | |

Furthermore, as for oral toxicity to mice, in the case of ribonucleic acid extracted from yeast, even a concentration of 5250 mg/kg has no effect, and therefore such ribonucleic acid has very low toxicity, namely, is extremely highly safe.
The daily intake of RNA and the like is generally 0.03 to 3.0 g, preferably 0.3 to 3 g.

The present invention uses one or more compounds selected from a group consisting of L-arginine, L-citrulline and L-ornithine as Component b).
Examples of preferred Component b) include L-arginine alone, L-citrulline alone or a mixture of L-arginirze and L-citrulline.
For L-arginine used in the present invention, each of naturally occurring compounds and synthetic compounds may be used, but the purity is preferably 97% or more.
L-arginine is one of the least toxic amino acids, so that the daily intake of L-arginine is optionally determined as necessary. The daily intake of L-arginine for an adult with a body weight of 70 kg is generally 0.5 to 9 g and preferably 0.5 to 6 g, but an amount of intake beyond the range may be used.
For L-citrulline used in the present invention, each of naturally occurring compounds and synthetic compounds may be used, but the purity is preferably 97% or more.
The daily intake of L-citrulline is optionally determined as necessary. The daily intake of L-citrulline for an adult with a body weight of 70 kg is generally 0.5 to 9 g and preferably 0.5 to 6 g, but an amount of intake beyond the range may be used.
For L-ornithine used in the present invention, each of naturally occurring compounds and synthetic compounds may be used, but the purity is preferably 97% or more.
The daily intake of L-ornithine is optionally determined as necessary. The daily intake of L-ornithine for an adult with a body weight of 70 kg is generally 0.5 to 9 g and preferably 0.5 to 6 g, but an amount of intake beyond the range may be used.
The total amount of daily intake of Component b) to be used is generally 0.5 to 9 g and preferably 0.5 to 6 g, but an amount of intake beyond the range may be used.
Increasing the amount of intake of Component b) is effective for treatment, but if the amount of intake of Component b) is too much, harmful NOₓ may be formed in the body. When excess NOₓ is formed in this manner, it is excreted in urine, so that an observation of the amount of NOₓ in urine can readily inhibit an excess dose of Component b). The observation of the amount of NOₓ in urine can be performed by quantitative determination of NO₂ and NO₃ or simply Tes-Tape. Namely, the administration of Component b) starts in a dose of 3 g/day at first. If a desired effect cannot be obtained, the dose is increased with observing the amount of NOₓ in urine, and then, if the amount of NOₓ increases, the dose is decreased. Here, the doses are in the case of oral administration.
The mass ratio of Component b) : Component a) is generally 1:0.006 to 6 and preferably 1:0.2 to 6.

For ascorbic acid as Component c) used in the present invention, each of naturally occurring compounds and synthetic compounds may be used, but the purity is preferably 97% or more. The toxicity of ascorbic acid is also extremely low.
As for the amount of ascorbic acid to be used, the mass ratio with respect to Component b) (Component b) : Component c)) is in the range of 1:0.2 to 20, specifically 1:0.2 to 6, for example, 1:0.2 to 0.25, 1:0.2 to 1/3, 1:0.2 to 1/2, 1:0.2 to 1, 1:0.2 to 4 or 1:0.2 to 6.
For most people, the optimum amount of intake of ascorbic acid is much higher than the upper limit of commonly recommended amount of intake. Each patient may select any amount of oral intake of ascorbic acid for good health maintenance. In this manner, the mass of ascorbic acid to be added in the mass ratio (Component b) : Component c)) of 1:0.2 to 0.25 described above is optional for intake. An upper limit of the mass ratio of Component c)/Component b) may be determined depending on the ratio of [upper limit of daily oral intake of Component c)/lower limit of daily intake of Component b)]. Generally, [upper limit of daily intake of Component c)=10 g (in the case of an adult with a body weight of about 70 kg))/lower limit of daily oral intake of Component b)=0.5 g (in the case of an adult with a body weight of about 70 kg))] can be 20, so that the upper limit of the mass ratio of Component c)/Component b) can be 20. Furthermore, the usual daily dose of Component c) (ascorbic acid) is said to be 2 to 3 g (corresponding to the mass ratio of Component c) (2 to 3 g)/Component b) (0.5 g)=4 to 6). Accordingly, Component b) : Component c) is 1:0.2 to 20, and preferably 1:0.2 to 6. L-arginine alone has a harsh taste and harsh feeling, but as far as it is used as a mixture in a range of the mass ratio described above, the harsh taste and harsh feeling of L-arginine becomes no problem.
In contrast, L-citrulline and L-ornithine have no harsh taste and harsh feeling like L-arginine.

Ascorbic acid has a tendency that lowers the level of uric acid in blood, which is increased by a large intake of RNA or the like. According to this, in the case of patients having a tendency that the level of uric acid is increased by the administration of RNA and the like, the symptoms of gout caused by the increased level of uric acid in blood can be avoided. Thus, ascorbic acid has an additional effect on avoiding the onset of gout.

Each form of Components while mixing and after mixing Components a) to c) is essential to be a solid, for example a powder, when containing L-arginine as Component b). It is not preferable that a mixed solution (aqueous solution) including Components a) to c) with Component b) containing L-arginine is prepared, because Maillard reaction occurs between both components of L-arginine and ascorbic acid to turn brown.
A mixed powder obtained from Components a) to c) with a powder of Component b) containing L-arginine shows little or no browning even at room temperature for 1 year.
Meanwhile, when Component b) without L-arginine (namely, L-citrulline or L-ornithine alone, or a mixture of two compounds of L-citrulline and L-ornithine) is used, an unfavorable reaction such as Maillard reaction does not occur, so that a mixed solution (aqueous solution) including Components a) to c) can be used.
Such a mixed solution (aqueous solution) can be preferably used as a liquid formulations (for example, a drinkable preparation) and as an intravenous drip solution.

The therapeutic agent for fibromyalgia syndrome or the therapeutic agent for pain caused by vascular smooth muscle spasm of the present invention has a tendency that the addition of a pharmaceutically acceptable calcium salt or magnesium salt to a mixture of Components a) to c) increases the pharmacological activity of the RNA and the like (Component a)). Examples of the pharmaceutically acceptable calcium salt and magnesium salt include compounds such as calcium lactate and magnesium sulfate in which the acid part is pharmacologically almost inactive and only the metal ion part is expected to be active. A mass ratio of the salt with respect to the total mass of RNA and the like depends on the mass of calcium or magnesium part, for example, calcium lactate is 1 to 5 times by mass, and magnesium sulfate is 1 to 5 times by mass.

When L-arginine is included as Component b), the addition of solid polyol such as a powder of xylitol or sorbitol, in addition to the additives mentioned above, is expected to further inhibit browning development by the reaction of arginine and ascorbic acid.

The therapeutic agent of the present invention may include DNA in addition to Components a) to c). Examples of the DNA include an extract derived from salmon soft roe.

Preferred aspects of the present invention include a therapeutic agent for fibromyalgia syndrome further containing the extract derived from salmon soft roe in addition to Components a) to c).

Furthermore, other preferred aspects include a therapeutic agent for pain caused by vascular smooth muscle spasm further containing the extract derived from salmon soft roe in addition to Components a) to c).

When a formulation is performed without water because Component b) includes L-arginine, the form of Components a) to c) and the additive described above is a solid suitable for mixing, for example, a powder to fine powder, crystals to fine crystals or a block readily pulverized into a fine powder or fine crystals. Furthermore, examples of the form of ascorbic acid (Component c)) also include a fine granule powder (for example, "'Vitamin Granule -97" manufactured by BASF Takeda Vitamins Ltd.) prepared by fluidized-bed granulation with an adhesive, for example, a small amount, for example, 3% of cornstarch.

Mixing of Components a) to c) and the additive when used is generally carried out by thoroughly stirring or pulverizing a powder to fine powder or crystals to fine crystals of each component so as to obtain a homogenous mixture. The mixing is generally accompanied with pulverization.

The mixing is carried out in a low humidity of 40% or below in order to minimize the oxidation of ascorbic acid in moisture.

A preferable mixing container has an inner surface of which material is inert to the components to be mixed and is a hard material, for example, ceramics such as earthenware. Examples of the mixing container include a mortar, mill or tube (cylinder or prism) made of ceramics such as earthenware and having smooth or rough inner surfaces and high durability. Ascorbic acid readily reacts with iron, and therefore, it is undesirable to use a mill type mixer having iron cutter blades for crushing.

The mixing is generally carried out at ordinary temperature, preferably 10 to 0.2°C, by thoroughly mixing or pulverizing so as to mix each component uniformly. Mixing time may be enough time to mix the components each other uniformly. A powder mixture after the completion of each component mixing generally has a particle size passing through a 50-mesh sieve, preferably a 100-mesh sieve, and even more preferably a 200-mesh sieve.
Furthermore, L-ascorbic acid may be coated with coating and then mixed with L-arginine. In this case, since L-ascorbic acid is covered with coating, L-ascorbic acid is not directly exposed to L-arginine, whereby no Maillard reaction occurs. As for L-ascorbic acid covered with coating, L-ascorbic acid completely coated with fat may be used.

The mixture obtained after mixing is used, if desired, with an additional component, without treatment (as a powder), or after formulating into tablets, pills, granules or capsules, as the therapeutic agent for fibromyalgia syndrome or the therapeutic agent for pain caused by vascular smooth muscle spasm. For example, the mixture may be mixed with one or two components properly selected from suitable pharmacologically acceptable binders (for example, gum arabic, sorbitol, gum tragacanth and polyvinyl pyrrolidone), diluents (for example, lactose, sugar, cornstarch, calcium phosphate, sorbitol and glycine), lubricants (for example, magnesium stearate, talc, polyethylene glycol and silica), disintegrators (for example, potato starch), aromatics or sweetening agents (for example, sugar, aspartame and saccharin) to form a powder, granules, tablets, capsules or the like.
Meanwhile, when Component b) without L-arginine (namely, L-citrulline or L-ornithine alone, or a mixture of two compounds of L-citrullinc and L-ornithine) is used, in addition to the formulations mentioned above, other formulations such as liquids (containing a drinkable preparation, suspension and syrup) may be adopted.

The therapeutic agent for fibromyalgia syndrome or the therapeutic agent for pain caused by vascular smooth muscle spasm of the present invention may be used in combination with other pharmaceutical drugs for treating the diseases such as an opioid antagonist, sodium-retaining agent/beta blocker, calcium-channel blocker/histamine blocker, antidepressant, antiallergic agent and anti acute anxiety agent.

The therapeutic agent for pain caused by vascular smooth muscle spasm of the present invention targets any blood vessels such as a brain blood vessel, lung blood vessel, blood vessels found in the intestines, so that the therapeutic agent can treat a pain of an organ with a disordered blood vessel, namely, the pain of the brain, lung, intestine, kidney, ureter or heart.
Furthermore, the pain caused by microangiopathy in Type II diabetes can be also targeted by the therapeutic agent for pain caused by vascular smooth muscle spasm of the present invention.

### [Example]

### (Preparation Example of RNA)

### Preparation Example 1. Preparation of RNA

(1) A slurry yeast recovered from a beer manufacturing process was passed through an 80- to 200-mesh sieve to remove a solid content, and washed with an aqueous solution of 0.5 to 1% sodium carbonate, and then with water to obtain debittered yeast.
(2) To the obtained yeast, sodium chloride and water were added so as to have a yeast concentration of 10% and a sodium chloride concentration of 10%, and the whole was heated gently and boiled for 2 to 5 hours. The boiling condition could be replaced by an autoclave treatment for 1 hour.
(3) Ribonucleic acid was extracted with boiling, then cooled, and subjected to solid-liquid separation by a centrifuge separator or the like to obtain an extraction liquid. As for residual RNA in the solid content, a 10% salt solution was added to the solid content to wash, and the washed solution was recovered to combine the extraction liquid.
(4) To the extraction liquid, concentrated hydrochloric acid was added to be pH 2 and a precipitated fraction under the acidic condition was obtained. The precipitated fraction was separated and recovered, and then sodium hydroxide was added to form the precipitate again. At the time, since the precipitate was formed immediately, the supernatant was removed and the precipitate was recovered by a centrifuge separator or the like. The obtained extract was dehydrated and washed with 98% ethanol, and then dried by an appropriate method.

### Preparation Example 2. Preparation of RNA

To 90 kg of dried brewer's yeast, 450 L of water was added, the whole was stirred for a predetermined period of time to solubilize impurities and to wash the yeast. Then, the washed yeast was recovered by centrifugation, and 800 L of water and 90 kg of sodium chloride were added and heated. After 2 hours boiling, an extraction liquid was obtained by a centrifuge separator. As for residual ribonucleic acid in the residue, a 10% salt solution was added and the washed solution was recovered. The washed solution was combined with the extraction liquid and the mixture was treated in the same manner as in (4) of Preparation Example 1 to obtain brewer's yeast crude RNA.

### Preparation Example 3. Preparation of RNA

A slurry brewer's yeast was debittered and washed in usual way, and then dried in a drum dryer at 120 to 140°C. To the dried brewer's yeast, 10 volumes of hot water at 95°C or higher was added and kept for 5 minutes, and then subjected to solid-liquid separation to obtain a solid. To the solid, sodium chloride and water were added to adjust a brewer's yeast solid concentration of 10% and a sodium chloride concentration of 10%, and then the whole was subjected to a homogenize at a discharge pressure of 600 kg/cm² or higher in order to break cell walls. The treated liquid containing the broken yeast was heated, boiled for 2 to 5 hours, and then treated in the same manner as in (3) and later of Preparation Example 1 to obtain brewer's yeast crude RNA.

Table 2 shows analysis results of the compositions of the crude RNA obtained in Preparation Examples.
[Table 2]

**Table 2**

| Composition/Preparation Example No. | No. 1 | No. 2 | No. 3 |
|---|---|---|---|
| Yield of Crude Ribonucleic Acid (%) | 1.51 | 2.57 | 3.09 |
| Component of Crude Ribonucleic Acid (%) | | | |
| CAS Fraction * | 3.0 | 6.0 | 5.4 |
| Ribonucleic Acid Fraction | 87.4 | 77.1 | 85.6 |
| DNA Fraction * | 0 | 1.2 | 1.32 |

| | | | |
|---|---|---|---|
| * Each component is a fraction analyzed by the Schmidt-Thannhauser-Schneider method (STS method) [Japan Society for Food Engineering, Food Analysis Method edited by the Committee of Editing Food Analysis Methods, p563, Korinsha (1982)], and CAS means acid soluble low molecular ribonucleic acids of nucleotide and nucleoside. | | | |

### (Acute Toxicity Test of Brewer's Yeast Crude RNA)

The brewer's yeast crude RNA obtained according to the method of Preparation Example 1 (an RNA content of 70%) was tested for oral acute toxicity (by forced administration) to mice (ICR strain, 40 mates and 40 females). Before administration, mice were fasted for 18 hours, the crude RNA was administrated at a dose of [7500 mg (converted to 5250 mg of RNA) suspended in 50 ml of water]/kg, and the subsequent 14 days observation revealed no abnormality.

### Preparation Example 4. Preparation of Soft Roe Extract

Two hundred and eleven grams of soft roe derived from salmon was broken, and then filtered to remove solid contents such as soft roe films. Then, to the filtrate, 1.5 1 of 0.14 mol/l salt solution was added, and the whole was ground and stirred to give an emulsion. From the emulsion, a supernatant was removed by centrifugation, and to the residue 1.5 1 of a 0.14 mol/l salt solution was added, then washed and filtered. The emulsification, centrifugation, washing and filtration were repeated twice or three times, and then the product was further washed with ethanol to remove ethanol-soluble organic substances and water, and dried under reduced pressure to obtain a soft roe extract in a powder form. The soft roe extract obtained in this manner is a light ash gray powder, and the data of chemical and physical properties of the extract are shown below. The ratio of nucleic acid and protein (W:W)=1.0:1.0 to 2.0: 1.0, a nucleic acid content: 25 to 50%, a protein content: 25 to 50%, an ash content: 5 to 15%, and ninhydrin reaction: positive.

### Example 1

In a ceramic mortar, 1 g of the brewer's yeast crude RNA powder (an RNA content of 70%) obtained according to the method of Preparation Example 1, 1 g of arginine powder and 0.2 g of ascorbic acid powder were placed, and the whole was thoroughly ground and mixed uniformly to obtain 2.2 g of a composition capable of passing through a #42 sieve (350 µ).

### Example of Pharmaceutical Formulation 1

One package contained 1.5 g of the powder mixture of brewer's yeast crude RNA, arginine and ascorbic acid obtained according to the method of Example 1.

### Example of Pharmaceutical Formulation 2

To 1.0 g of the powder mixture of brewer's yeast crude RNA, arginine and ascorbic acid obtained according to the method of Preparation Example 1, 3.0 g of sodium ascorbate was mixed to make one package.

### Example of Pharmaceutical Formulation 3

To 1.0 g of the powder mixture of brewer's yeast crude RNA, arginine and ascorbic acid obtained according to the method of Preparation Example 1, 3.0 g of sodium ascorbate, 1.0 g of calcium lactate and 1.0 g of magnesium sulfate were mixed to make one package.

### Example of Pharmaceutical Formulation 4

To 1.0 g of the brewer's yeast crude RNA obtained according to the method of Preparation Example 1, 1.0 g of calcium lactate and 1.0 g of magnesium sulfate were mixed to make one package.

### Example of Pharmaceutical Formulation 5

To 1.5 g of the powder mixture of brewer's yeast crude RNA, arginine and ascorbic acid obtained according to the method of Preparation Example 1, 2 g of the extract derived from salmon soft roe (prepared according to Preparation Example of Soft Roe Extract) and 4 g of ascorbic acid powder were added and mixed to make one package.

### Clinical Example I

A 43-year-old female (homemaker) had the history of generalized myalgia from several years ago and became immobile from 1980 because the pain became gradually stronger. During that time, she visited various medical institutions to be treated with nerve block (epidural nerve block), but no effect was shown, so that she had to stay in bed at home.
Hearing a rumor of our hospital, her family member visited the outpatient department. The patient was supposed to suffer from "fibromyalgia syndrome", so that the composition produced in Example 1 was prescribed as a single-dose of 20 g for 7 days. If the patient was the disease, she would become mobile by the prescription.
The pain especially along the carotid artery and spine became mild and she became ambulatory, so that the patient herself visited the hospital. The pain at visit remained only tenderness of the thigh side and she could walk.
For 20 days, she had taken the composition produced in Example 1 in a dose of 20 g/day, and the pain became further mild. From after 3 weeks, she orally took it every other day and she seemed to recover completely after 3 months. Then, she had led a normal life since recovery, but after 3 months, the treatment was restarted because of the sign of recurrence, and she continued to take the composition produced in Example 1.

### Clinical Example 2

A 70-year-old female (homemaker) had been healthy by nature, had a back pain for one year but led a normal life without special problems. After 3 months, the right blepharism occurred without pain, and the right visual acuity on which she had depended became inconvenient. She visited a certain famous eye clinic and got an injection of botulinum toxin. The treatment was extremely effective, so that, after that, she visited the outpatient department every 3 months and continued the botulinum toxin injection. After a while, she had not been worried about the back pain because of the attention to the right blepharism till then, but became strongly aware of the back pain suddenly.
She visited the outpatient department of our hospital, having tenderness along the spine in the chest and back regions in spite of the botulinum injection. Tender points in the thigh region proposed by Prof. Nishioka, St. Marianna University School of Medicine, were confirmed, so that it was supposed to be fibromyalgia syndrome, and 3000 mg of the composition produced in Example 1 and 6000 mg of vitamin C were orally administered. After 3 weeks, the back pain became mild, As for the thigh side pain, only few tender points remained.
The composition produced in Example 1 was administered every other day.
As shown in the example, a local injection of a trace amount of botulinum toxin is effective for the eyelid, but has no effect on generalized myalgia.
After the oral administration of the composition produced in Example 1 started, the eyelid injection every 3 months was stopped, but the botulinum became unnecessary and the progress was good.
The result of Clinical Example 2 shows that the composition pertaining to the present invention improved muscle spasm. Furthermore, the composition pertaining to the present invention improved cardiac muscle abnormality, so that the composition is predicted to also prevent acute atrial fibrillation and ventricular fibrillation.

### Clinical Example 3

A 45-year-old female (homemaker) had a history of stiff shoulders from 20 years earlier with cervical headache (occipital headache). Various treatments gave only temporary effect. She did not have any pain in her arms, so that it was not cervicobrachial syndrome. She had no tender points in thorax and abdomen, but strong tenderness was confirmed outside of the thigh of the leg. It was diagnosed as one symptom of fibromyalgia syndrome, so that the administration of the composition produced in Example 1 and vitamin C (containing 3000 mg of arginine, 2000 mg of vitamin C and RNA) was started. As a result, only after 3 days, the cervical headache became mild at the outpatient department. The thigh tenderness remained but became considerably weak. After about 3 weeks, the tenderness in this region disappeared. Then, the administration of the composition produced in Example 1 and vitamin C was stopped and the pain disappeared for a while. However, since mild stiff shoulders started to appear after 2 to 3 weeks, the administration (3 g of arginine and others) was carried out once again, and then the stiff shoulders disappeared at once. Thus, the administration was carried out every 2 or 3 days and the amount of arginine kept 3000 mg (3 g).

### Clinical Example 4.

A 66-year-old male, a large company president, with a height of 170 cm and a body weight of 70 kg, has been diagnosed as diabetes mellitus (hereinafter referred to as DM) for more than 10 years. Because of anosognosia, he seemed to pay attention to eating, but actually not. Recently, he had arrhythmia and anginal pain like attacks, so that he visited a hospital. At the outpatient department, it was confirmed that his arrhythmia was accompanied with mild coronary insufficiency due to atrial fibrillation, from an electrocardiogram. He refused to be admitted to hospital for thorough checkup because of busy schedule. Vascular examinations of the brain, heart and neck by MRI showed mild coarctation of anterior descending coronary artery but no atheromatous degeneration. The pain was diagnosed to be probably an anginal pain due to vascular spasm.
Three thousand milligrams of the composition produced in Example 1 and 6000 mg of vitamin C were administered, and as a result, the arrhythmia and anginal pain like attacks did not occur at all.

Organ pain due to vascular smooth muscle spasm as in Clinical Example 4 is considered that fibromyalgia syndrome occurs in a visceral organ. Thus, the composition pertaining to the present invention is supposed to have an excellent effect on the visceral organs as well as the fibromyalgia syndrome.
The composition pertaining to the present invention is supposed to have the excellent effect, because the organ pain due to vascular disorder is supposed to be caused by the spasm of smooth muscle completely found in the blood vessel.
From the result with respect to the anginal pain in Clinical Example 4, it is suggested that the composition pertaining to the present invention would also have effects on various pains in other organs such as the brain, lung, intestines, kidney, ureter and heart as well as a pain derived from vascular disorder due to DM.

The smooth muscle such as artery is supposed to be dilated by the composition pertaining to the present invention, so that generalized arterioles are dilated. Then, the circulations of not only brain blood vessel, lung blood vessel and blood vessel found in the intestines but also the renal circulation become good, so that the composition is expected to prevent and treat diseases related to these organs, for example, cerebral infarction, pulmonary hypertension, angina pectoris and myocardial infarction related to the coronary artery, and intestinal necrosis due to intestine artery hematogenous disorder.
In addition, the composition pertaining to the present invention is excellent because of no adverse effect.

## Claims

1. A therapeutic agent for fibromyalgia syndrome, comprising:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid,
wherein the mass ratio of Component b) : Component c) is 1:0.2 to 20.

2. The therapeutic agent for fibromyalgia syndrome according to claim 1, wherein the mass ratio of Component b) : Component c) is 1:0.2 to 6.

3. The therapeutic agent for fibromyalgia syndrome according to claim 2, wherein the mass ratio is 1:0.2 to 0.25.

4. The therapeutic agent for fibromyalgia syndrome according to any one of claims 1 to 3. wherein one or more compounds selected from the group consisting of RNA, ribonucleotide and ribonucleoside are an extract(s) from yeast.

5. The therapeutic agent for fibromyalgia syndrome according to claim 4, wherein the yeast is a brewer's yeast.

6. The therapeutic agent for fibromyalgia syndrome according to any one of claims 1 to 5, further comprising an extract derived from salmon soft roe.

7. A therapeutic agent for pain caused by vascular smooth muscle spasm, comprising:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid,
wherein the mass ratio of Component b) : Component c) is 1:0.2 to 20.

8. The therapeutic agent for pain caused by vascular smooth muscle spasm according to claim 7, wherein the mass ratio of Component b) : Component c) is 1:0.2 to 6.

9. The therapeutic agent for pain caused by vascular smooth muscle spasm according to claim 8, wherein the mass ratio is 1:0.2 to 0.25.

10. The therapeutic agent for pain caused by vascular smooth muscle spasm according to any one of claims 7 to 9, wherein one or more compounds selected from the group consisting of RNA, ribonucleotide and ribonucleoside are an extract(s) from yeast.

11. The therapeutic agent for pain caused by vascular smooth muscle spasm according to claim 10, wherein the yeast is a brewer's yeast.

12. The therapeutic agent for pain caused by vascular smooth muscle spasm according to any one of claims 7 to 11, further comprising an extract derived from salmon soft roe.

13. Use, for producing a therapeutic agent for fibromyalgia syndrome, of a composition comprising:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid,
wherein the mass ratio of Component b) : Component c) is 1:0.2 to 20.

14. A method for treating fibromyalgia syndrome, comprising:
administering a therapeutically effective amount of a composition including:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid
to a patient requiring the treatment,
wherein the mass ratio of Component b) : Component c) is 1:0.2 to 20.

15. Use, for producing a therapeutic agent for pain caused by vascular smooth muscle spasm, of a composition comprising:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid,
wherein the mass ratio of Component b) : Component c) is 1:0.2 to 20.

16. A method for treating pain caused by vascular smooth muscle spasm comprising:
administering a therapeutically effective amount of a composition including:
a) one or more compounds selected from a group consisting of RNA, ribonucleotide and ribonucleoside,
b) one or more compounds selected from a group consisting of L-arginine, L-citrulline and L-ornithine, and
c) L-ascorbic acid
to a patient requiring the treatment,
wherein the mass ratio of Component b): Component c) is 1: 0.2 to 20.
